(19) Europäisches Patentamt
European Patent Office
Office européen des brevets

(11) Publication number: 0 292 170
A2

(12) # EUROPEAN PATENT APPLICATION

(21) Application number: 88304174.1

(22) Date of filing: 09.05.88

(51) Int. Cl.4: G01N 33/58 , G01N 33/535 , //G01N33/542,G01N33/94

(30) Priority: 18.05.87 US 50349

(43) Date of publication of application: 23.11.88 Bulletin 88/47

(84) Designated Contracting States: BE CH DE ES FR GB IT LI NL SE

(71) Applicant: TECHNICON INSTRUMENTS CORPORATION
511 Benedict Avenue
Tarrytown, New York 10591(US)

(72) Inventor: Adolfsen, Robert H.
52 Coachlight Square
Montrose New York 10548(US)
Inventor: Saini, Mohan S.
27 Harmony Road
Spring Valley New York 10977(US)
Inventor: Vunnam, Ranga R.
36 Bellwood Drive
New City New York 10956(US)

(74) Representative: Wain, Christopher Paul et al
A.A. THORNTON & CO. Northumberland House 303-306 High Holborn
London WC1V 7LE(GB)

(54) **Enzyme immunoassay.**

(57) A test composition for use in a specific binding enzyme immunoassay for detecting an analyte in a sample, includes: (a) a target protein capable of mediating a signal generating process, the mediating activity of the target protein being changed by covalently bonding a regulatory group thereto; (b) a regulatory enzyme capable of catalyzing the covalent bonding of the regulatory group to the target protein; (c) a conjugate of a substrate molecule for the regulatory enzyme and an analyte, the substrate containing the regulatory group; (d) a specific binding partner for the analyte; and (e) reagent means for generating a detectable signal responsive to the condition of the target protein.

EP 0 292 170 A2

Xerox Copy Centre

## ENZYME IMMUNOASSAY

This invention relates to a test composition and method for performing an enzyme immunoassay.

The development of specific binding assay techniques has provided extremely useful analytical methods for determining various organic substances of diagnostic, medical and environmental importance which appear in liquid media at very low concentration. Specific binding assays are based on the specific interaction between a bindable analyte under determination, and a binding partner therefor.

In a conventional, non-isotopic competitive binding assay, a reagent means is provided which includes: (1) a labeled conjugate in the form of the analyte to be detected (e.g. an antigen or hapten) which constitutes the binding component of the conjugate chemically linked to a label component; and (2) a specific binding partner for the analyte (e.g. an antibody). Upon the combination of the test sample, and the reagent means, the analyte to be detected in the test sample, if present, and the binding component of the labeled conjugate compete in a substantially non-discriminating manner for non-covalent binding sites on the specific binding partner. The amount of labeled conjugate bound to the binding partner (i.e. the bound-species) or the amount which remains not bound to the binding partner (i.e. the free-species) is a function of the amount of analyte present in the test sample. Therefore, the amount of analyte in the test sample can be determined by measuring the amount of labeled conjugate resulting in either species and comparing the amount so determined with standard results obtained using known concentrations of analyte.

Where the labeled conjugate in the bound-species and that in the free species are essentially indistinguishable by the means used to monitor the label component, the bound-species and the free-species must be physically separated in order to complete the assay. This type of assay is referred to in the art as "heterogeneous". Where the bound-species and free-species can be distinguished, a "homogeneous" format may be followed and the separation step avoided.

The first discovered type of highly sensitive specific binding assay was the radioimmunoassay which employs a radioactive isotope as the label component. Such an assay necessarily must follow the heterogeneous format since the monitorable character of the label is qualitatively unchanged in the free and bound-species. Because of the inconvenience and difficulty of handling radioactive materials, many new assay systems have been devised using materials other than radioisotopes as the label component, including enzymes, fluorescent molecules, bacteriophages, metals and organometallic complexes, coenzymes, enzymes substrates, enzyme inhibitors, cyclic reactants, and chemiluminescent reactants.

Exemplary of methods which have been developed using an enzyme as the labeling substance are those described in U. S. Patent Nos. 3,654,090; 3,791,932; 3,839,153; 3,850,752; and 3,879,262 and in the Journal of Immunological Methods 1:247 (1972) and the Journal of Immunology 109:129 (1972). In each of the described methods an enzyme is chemically coupled to either the ligand to be detected or a binding partner thereof and an appropriate heterogeneous specific binding reaction scheme is constructed whereby after incubation with a sample, the amount of enzymatic activity associated with either the insoluble portion or the liquid portion is a function of the amount of ligand in the sample. The problems associated with the synthesis, characterization, and stability of the enzyme-conjugates are serious short-comings of this approach.

An enzyme-labeled immunoassay of the homogeneous type is described in U. S. Patent No. 3,817,834 wherein a ligand enzyme conjugate is employed. The enzymatic activity of the conjugate in the bound-species is measurably less than that in the free-species thereby allowing a homogeneous format to be used. The use of particularly unique materials as labeling substances, including chemiluminescent molecules, cyclic reactants, and cleavable fluorescent enzyme substances in both homogeneous and heterogeneous formats is described in German Offenlegungschriften Nos. 2,618,419 and 2,618,511 based on U. S. patent application serial numbers 667,982 and 667,996, filed on March 18, 1976, and assigned to Miles Laboratories, Inc.

British Patent No. 1,392,403 and French Patent No. 2,201,299, which correspond to U. S. Patent No. 3,880,934, describe a specific binding assay which utilizes a non-active precursor of a spectrophotometrically-active substance as the labeling substance. After incubation of the sample with the specific binding reaction system, the insoluble and liquid portions are separated and the amount of labeling substance present in the liquid portion, which is a function of the amount of ligand to be detected in the sample, is determined by carrying out reaction steps that transform the active labeling substance into a chromogen or fluorometrically active material which is then measured by conventional means.

Other specific binding assay methods employing different types of labeling substances are disclosed in: U. S. Patent No. 3,850,578 which discloses the use of electron spin resonance as a labeling means; U. S. Patent No. 3,901,654 which discloses the use of fluoroescense quenching and enhancement as a labeling

means; and Report No. PB-224,875 of the National Technical Information Service (NTIS) of the U. S. Department of Commerce (1973) which describes an unsuccessful attempt to use hemin chloride as a labeling substance in a heterogeneous assay system monitored by a chemiluminescence reaction. Nature 219:186 (1986) describes in detail certain radioimmunoassay procedures and makes a passing reference of a very general nature to the possible use of coenzymes and viruses in place of radioisotopes as labeling substances. However, the author provides no enlightenment as to how to carry out an assay using such alternative labeling substances, or in fact as to whether such an assay would be operable. For further background, reference may be had to Principles of Competitive Protein-Binding Assays, ed. Odell and Daughaday (J. B. Lippincott Co., Philadelphia, 1972) which discusses in breadth the various known assay schemes and the different materials and features that have been used as labels for specific binding assays.

Even through many new types of specific binding assays have been suggested and investigated, the radioimmunoassay and the various enzyme-tagged immunoassays remain the most widely used and improved. However, both types of systems have obvious shortcomings, the radioimmunoassay in the use of radioactive material which is hazardous and which requires careful handling and the enzyme-tagged immunoassays in the difficulty of preparing useful enzyme-tagged conjugates which remain stable.

U. S. Patent No. 4,134,792 describes a specific binding assay method of employing, as a labeling substance, a reversibly binding enzyme modulator that may be used in both homogeneous and heterogeneous binding assay formats wherein the liquid medium to be assayed for a ligand is combined with a reagent comprising a labeled conjugate to form a binding reaction system having a bound-species and a free-species of the conjugate. The distribution of the conjugate between the bound-species and the free-species is determined by addition of an enzyme whose activity is affected, either in an inhibitory (e.g., competitive inhibitor) or stimulatory (e.g., allosteric effector) manner, by said modulator and measuring the resulting activity of the enzyme.

U. S. Patent No. 4,273,866 describes a method for determining ligands in test samples comprising intermixing with the test sample a ligand analog-irreversible enzyme inhibitor conjugate and a binding protein bindable to the ligand and the ligand analog-irreversible enzyme inhibitor conjugate and wherein the amount of ligand analog-irreversible enzyme inhibitor conjugate bound by the binding protein is related to the amount of ligand in the test sample, said binding protein inactivating the irreversible enzyme inhibitor when bound to the ligand analog portion of the conjugate; intermixing an enzyme which is irreversibly inhibited by the ligand analog-irreversible enzyme inhibitor conjugate unbound by the binding protein; and intermixing substrate to the enzyme and monitoring the enzyme substrate reaction.

U. S. Patent No. 4,230,797 describes a heterogeneous specific binding assay method and means based on the use of a labeling substance and reactant substance which exhibits reactant activity as a constituent of a predetermined reaction system. The amount of the reactant present in either of the bound- and free-phase is determined by contacting either phase with at least one reagent which forms, with the reactant, the predetermined reaction system which serves as a means for monitoring the specific binding reaction. The method requires a separation step of the bound-phase and free-phase.

U. S. Patent No. 4,279,992 describes a method and reagent for determining a ligand in a liquid medium employing, as an enzyme-cleavable substrate label, a residue having the formula

G-D-R

wherein G is a glycone, D is a dye indicator moiety, and R is a linking group through which the label residue is covalently bound to a binding component of a conventional binding assay system, such as the ligand, an analog thereof, or a specific binding partner thereof. The monitored characteristic of the label is the release of a detectable product, usually a fluorogen or chromogen, upon enzymatic cleavage of the glycosidic linkage between the glycone and the dye indicator moiety. The assay method may follow a homogeneous or heterogeneous format.

U. S. Patent No. 4,238,565 describes an assay for determining a ligand in a liquid medium employing an organic prosthetic group residue, such as a residue of flavin adenine dinucleotide, flavin mononucleotide, or heme, as a label component in the labeled conjugate. Preferably, the label component is the prosthetic group residue alone or is a holoenzyme residue comprising such prosthetic group residue combined with an apoenzyme in the form of a holoenzyme complex. In the former case, the label component preferably is monitored in the assay by adding an apoenzyme after the binding reaction has been initiated and measuring the resultant holoenzyme activity. In the latter case, the label component is monitored simply by measuring holoenzyme activity. The assay method may follow conventional homogeneous and heterogeneous schemes.

U. S. Patent No. 4,463,090 describes enzyme immunoassays whose sensitivity is increased by cascade amplification. The coupled ligand (enzyme or an activator) catalytically activates a second enzyme which acts on a substrate or can act on a third enzyme to produce a cascade.

"Zymogen Activation: A New System for Homogeneous Immunoassay" Clin. Chem., 30, pp. 1452-1456 (1984) describes a three-step cascade reaction in which blood clotting Factor X conjugated to an antigen is converted into an active form after a competitive binding process. A peptide is cleaved off the molecule to convert the inactive form into an active form.

In accordance with the present invention, enzyme immunoassays are provided in which a regulatory enzyme covalently modifies a target protein thereby altering the rate of a detectable signal generating process mediated by the target protein. The degree of modification is a function of the amount of analyte in the test sample.

The invention provides a test composition which includes a target protein capable of mediating a signal generating process. The mediating activity of the target protein is modified if a regulatory group is covalently bonded thereto. Also included in the composition is a regulatory enzyme capable of catalyzing the covalent bonding of the regulatory group to the target protein. A conjugate of a substrate molecule for the regulatory enzyme and the analyte is provided, along with a specific binding partner for the analyte, and reagent means responsive to the condition of the target protein for generating a detectable signal.

The invention further provides a method detecting the presence and amount of an analyte in a sample which method consists essentially of the steps of: (a) providing a regulatory enzyme and a target protein, the target protein being capable of mediating a process which yields a detectable signal, and the regulatory enzyme being capable of catalyzing the covalent bonding of a regulatory group to the target protein whereby the rate of the process mediated by the target protein is altered; (b) providing a conjugate comprising the analyte covalently bonded to a substrate for the regulatory enzyme which contains the regulatory group; (c) providing a specific binding partner of the analyte which is capable of binding to the conjugate restricting the conjugate from acting as a substrate for the regulatory enzyme; (d) contacting the conjugate with the specific binding partner in the presence of the regulatory enzyme, the target protein, the elements of the process mediated by the target protein, and the sample; (e) measuring the rate of the process mediated by the target protein; and (f) comparing the rate of the process conducted in the presence of the sample with the rate of the process when conducted in the presence of a series of standard compositions containing known amounts of the analyte.

The assays of this invention may be used for the qualitative and/or quantitative measurement of any substance which may be assayed using an antibody-antigen reaction, i.e. an immunoassay. In contrast to conventional enzyme-linked immunoassays, the present assay uses a regulatory enzyme-target protein pair. The regulatory enzyme is adapted to catalyze the covalent bonding of a regulatory group from the substrate to the target protein which results in the the change in the ability of the target protein to mediate the activity of a signal generating process. The regulatory enzyme transfers part of a substrate molecule of a conjugate to a specific site on the target protein separate and distinct from the active site. It is to be understood that this type of enzyme activity regulation is not allosteric. Allosteric regulation involves the noncovalent binding of a regulatory molecule at a site separate and distinct from the active site. Further, a regulatory enzyme is not involved in such regulation.

In the following discussion, examples of suitable regulatory enzymes and target proteins and specific regulatory enzyme-target protein pairs will be given, followed thereafter by examples illustrative of the present invention.

Some representative examples of regulatory enzymes are the protein kinases listed in Table I (E. G. Krebs and J. A. Beavo, Ann. Rev. Biochem., 48, 923-959 (1979); C. S. Rubin and O. M. Rosen, Ann. Rev. Biochem., 44, 831-887 (1974)). The use of protein kinases as regulatory enzymes results in protein phosphorylation whereby regulation of the target protein is achieved by the transferring of a phosphate group from a conjugate substrate to the target protein. In addition to protein phosphorylation, regulation may be achieved by attaching other types of moieties covalently to the target protein. For example, adenylation is a means of regulating E. coli glutamine synthetase and E. coli lysine sensitive aspartyl kinase (E. R. Stadtman, The Enzymes, VIII, 1-49 (1973)). Regulation may also be achieved by uridylation, acetylation or methylation (Krebs and Beavo, ibid).

TABLE 1

EXAMPLES OF REGULATORY ENZYMES (PROTEIN KINASES)

cAMP Dependent Protein Kinase
cGMP Dependent Protein Kinase
cIMP Dependent Protein Kinase
Phosphorylase Kinase, Ca $_{2}^{+}$ dependent
Myosin Light Chain Kinase, Ca $_{2}^{+}$ dependent
Double Stranded RNA Dependent Protein Kinase
Pyruvate Dehydrogenase Kinase
Membrane Protein Kinase
Histone Protein Kinase
Ribosomal Protein Kinase

Examples of target proteins appear in Table 2 and include those enzymes which are phosphorylated by protein kinases (Krebs and Beavo, ibid; Rubin and Rosen, ibid). Some of these enzymes are themselves protein kinases. Also, the target proteins do not necessarily need to be cytoplasmic enzymes. Membrane proteins can be phosphorylated to result in changes in membrane permeability. For example, synaptic membranes are known to be phosphorylated by a cIMP dependent kinase. In addition, ribosomal proteins can be phosporylated by a cAMP dependent kinase, possibly resulting in regulation of protein synthesis. Histones can be phosphorylated, resulting in regulation of gene expression.

TABLE 2

TARGET ENZYMES FOR PROTEIN KINASES

Acetyl CoA Carboxylase
Cholesterol Ester Hydrolase
DNA-Dependent RNA Synthetase
Fructose 1, 6 Diphosphatase
cGMP Dependent Protein Kinase
Glycerophosphate Acyltransferase
Glycogen Phosphorylase
Glycogen Synthetase
Hormone Sensitive Lipase
Hydroxymethylglutaryl CoA Reductase
Myosin light chain
NAD Dependent Glutamate Dehydrogenase (Yeast)
Phenylalanine Hydroxylase
Phosphofructokinase (liver)
Phosphorylase Kinase
Phosphorylase Phosphatase Inhibitor
Polynucleotide Phosphorylase
Pyruvate Dehydrogenase
Pyruvate Kinase (liver)
Reverse Transcriptase
Tryptophan Hydroxylase
Type II cAMP Dependent Protein Kinase
Tyrosine Hydroxylase

The preferred regulatory enzyme-target protein pair of the present invention is the phosphorylase kinase system, in which phosphorylase kinase is the regulatory enzyme and glycogen phosphorylase (phosphorylase b) is the target. The terminal phosphate of an ATP-conjugate is transferred to phosphorylase b (inactive form) to convert it into phosphorylase a (active form) by the regulatory enzyme, phosphorylase kinase. This phosphorylation process greatly increases the activity of the target enzyme.

Another preferred system is the glycogen synthetase system. In this case, the phosphorylated form of the target enzyme (glycogen synthetase) has a lower activity than the unmodified enzyme. The regulatory enzyme is cyclic AMP-dependent protein kinase.

An example in which some group other than phosphate is attached is that of glutamine synthetase. The regulatory enzyme is adenyltransferase. Adenyltransferase attaches an adenyl group to glutamine synthetase (target). Upon attachment of the adenyl group, the activity of this enzyme declines.

Further description of these enzymes may be found in H. Holzer and W. Duntze, Ann. Rev. Biochem., 40, 756 (1971). Additional references discussing these and other similar systems include the following: H. L. Segal, Science, 180, 25 (1973); D. H. Brown and C. F. Cori, The Enzymes, 5, 207 (1961); P. B. Chock and E. R. Stadtman, Method in Enzymol., 64, 297 (1980).

In the traditional heterogeneous assay format, an antibody is mixed with a specimen containing the analyte and the conjugate which contains the enzyme substrate (such as adenosine triphosphate "ATP") covalently attached to the analyte. Then a separation of free conjugate from bound conjugate is effected by conventional procedures. Free conjugate is then assayed by incubation with the enzyme pair, the result of which is an alteration of the activity of the target protein. The activity of the target protein is then measured. If all components are soluble, separation of free conjugate is effected by filtration (for example, using Amicon ultrafiltration protein concentrators or gel filtration columns). Or, an agent is added which precipitates antibody-analyte and antibody-conjugate complexes, such as anti-antibody. Or, the antibody is immobilized on a tube or a microtiter plate, and the solution containing free conjugate is physically transferred to a separate tube.

The incubation with antibody is carried out either in a competitive or sequential manner. In the latter case, the antibody is incubated with the specimen prior to the addition of the conjugate, which may improve sensitivity somewhat. It should be understood that these are just a few of many possible ways in which heterogeneous assays can be carried out using this concept.

In a homogeneous assay, separation of free and bound conjugate is not necessary. For example, if the antibody prevents bound conjugate from acting as a substrate for the regulatory enzyme, then no separation is required. The assay protocol is either competitive or sequential. In the latter case, the antibody is incubated with the specimen before the conjugate is added, which may improve sensitivity somewhat.

Generally, the homogeneous enzyme immunoassay in accordance with the present invention involves the use of a target protein which is capable of mediating a process that yields a detectable signal. By the term "detectable signal" is means a change in or appearance of a property in the enzyme system which is capable of being perceived, either by direct observation or instrumentally, and which is a function of the presence of the analyte in the sample. Some examples of detectable signals are changes in visible or infrared absorption fluorecsence, phosphorescence, reflectance or chemiluminescence. Other examples of detectable signals may be the change in electrochemical properties.

The concepts of the present invention may be utilized in either a heterogeneous or homogeneous format as desired.

The immunoassay of the present invention includes a conventional competitive binding equilibrium between: (a) the conjugate of a substrate molecule and the analyte to be detected; and (b) a specific binding partner for the analyte. This binding equilibrium occurs in the presence of a regulatory enzyme which is capable of catalyzing the covalent bonding of a regulatory group in the substrate to a target protein. The target protein is capable of mediating, e.g., stimulating or inhibiting, a detectable signal generating process when a regulatory group is covalently bonded thereto. The change in the detectable signal is then measured and the quantity of analyte in the test sample can be determined.

One preferred embodiment includes: (a) a competitive binding equilibrium involving a conjugate containing ATP (I)

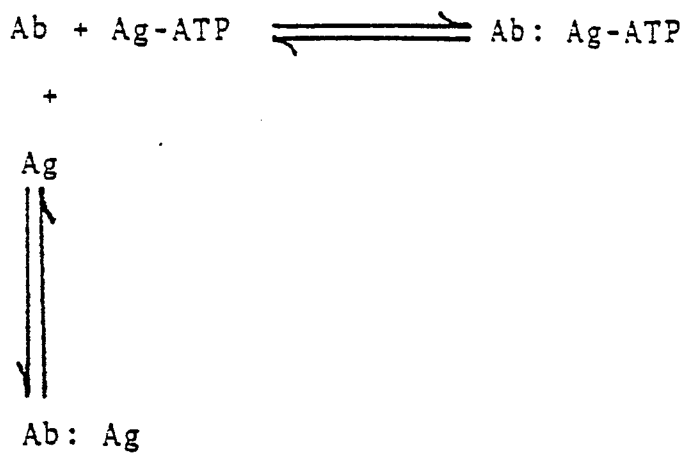


(I)

(b) a catalyst transformation step (II) employing as the regulatory enzyme phosphorylase kinase and as the target protein inactive phosphorylase b, wherein the terminal phosphate of the ATP moiety of the conjugate is transferred to inactive phosphorylase b to convert inactive phosphorylase b to active phosphorylase a

Ag-ATP + Phos b → Ag-ADP + Phos a (II)

(c) a detectable signal generation sequence wherein: (i) active phosphorylase a produces a first signal precursor, glucose 1-phosphate from glycogen and inorganic phosphate ($P_i$); (ii) phosphoglucomutase produces as second signal precursor glucose 6-phosphate from glucose 1-phosphate; (iii) glucose 6-phosphate dehydrogenase produces the reduced form of nicotinamide adenine dinucleotide phosphate (NADPH) and 6-phosphogluconate from glucose 6-phosphate and nicotinamide adenine dinucleotide phosphate (NADP) and (iv) the rate of NADPH production is then measured.

$$\text{Glycogen} + P_i \underset{}{\overset{\text{Phos. a}}{\rightleftharpoons}} \text{Glucose 1-phosphate}$$

$$\text{Glucose 1-phosphate} \overset{\text{Phosphoglucomutase}}{\rightleftharpoons} \text{Glucose 6-phosphate}$$

$$\text{Glucose 6-phosphate} + \text{NADP} \overset{\text{glucose 6-phosphate dehydrogenase}}{\rightleftharpoons} \text{6-phosphogluconate} + \text{NADPH}$$

In the above sequence, one free conjugate produces one active enzyme, and the amplification factor of the assay should be equivalent to the turnover number of phosphorylase a. This should result in an assay of high sensitivity.

There are two basic requirements that the conjugate used in this embodiment of the present invention must fulfill. First, it should be a substrate for phosphorylase kinase. Second, it should not be a substrate for the enzyme when it is bound to the antibody. For example, an ATP-dinitrophenyl conjugate was found to be a substrate of luciferase when it was free in solution but not when bound to the antibody (Carrico, R. J. et al. Anal. Biochem., 76, pp 95-110 (1976)).

A second preferred immunoassay embodiment utilizes branched chain ketoacid dehydrogenase (BCKDH) as the target enzyme. The regulatory enzyme is BCKDH kinase. After the competitive binding reaction, (I) above, free conjugate is utilized by said kinase to phosphorylate said target protein:

$$\text{Ag-ATP} + \text{BCKDH} \xrightarrow{\text{BCKDH Kinase}} \text{Ag-ADP} + \text{BCKDH-P}$$

In this embodiment, phosphorylation inhibits the activity of the target enzyme by about 99%. The rate of nicotinamide adenine dinucleotide (NADH) production in the following reaction would, therefore, be inversely proportional to the amount of analyte in said test sample. The reaction which is mediated by the target protein is the following:

$$\text{BCK} + \text{NAD} + \text{CoASH} \xrightarrow[\text{TPP, } Mg^{2+}]{\text{BCKDH}} \text{BCK-CoA} + CO_2 + \text{NADH}$$

where BCK is branched chain ketoacid, CoASH is the reduced form of coenzyme A, and TPP is a cofactor, thiamin pyrophosphate. This system is described in more detail by P. J. Randle, P. A. Patston, and J. Espinal in The Enzymes, XVIII, 97 (1987).

A third preferred embodiment utilizes hormone sensitive lipase (HSL) as the target protein. The regulatory enzyme is cAMP dependent protein kinase. After the competitive binding reaction, free conjugate is utilized by the kinase to phosphorylate the target protein, resulting in a three-fold increase in activity:

$$\text{Ag-ATP} + \text{HSL} \xrightarrow{\text{cAMP-dependent protein kinase}} \text{Ag-ADP} + \text{HSL-P}$$

The reaction mediated by the target protein is as shown below, with two additional enzymes being required as coupling enzymes to convert intermediate signal precursors into the final signal generating material, NADH:

$$\begin{array}{c}\text{Triacylglycerol}\\ \text{(or Diacylglycerol)}\end{array} \xrightarrow{\text{HSL-P}} \begin{array}{c}\text{Diacylglycerol}\\ \text{(or monoacylglycerol)}\end{array}$$

$$\text{Monoacylglycerol} \xrightarrow[\text{Lipase}]{\text{Monoacylglycerol}} \text{Fatty Acid} + \text{Glycerol}$$

$$\text{Glycerol} + \text{NAD} \xrightarrow{\text{Glycerol Dehydrogenase}} \text{Dihydroxyacetone} + \text{NADH}$$

This system is described in detail by P. Stralfors, H. Olsson and P. Belfrage in The Enzymes, XVIII, 147 (1987).

A further preferred embodiment utilizes hydroxymethylglutaryl-CoA reductase (HMGCR) as the target protein and HMGCR kinase as the regulatory enzyme. After the competitive binding reaction, free conjugate is utilized by the HMGCR kinase to phosphorylate the target protein, as follows:

$$\text{Ag-ATP} + \text{HMGCR} \xrightarrow{\text{HMGCR kinase}} \text{Ag-ADP} + \text{HMGCR-P}$$

In this system, phosphorylation substantially inhibits the activity of the target protein. Therefore, the rate of the enzyme reaction would be inversely related to the amount of analyte in the test sample. The reaction

mediated by the target protein is:

$$\text{HMG-CoA} + 2\ \text{NADPH} \xrightarrow{\text{HMGCR}} \text{Mevalonate} + \text{CoASH} + \text{NADP}$$

As indicated in the above reaction scheme, it is the disappearance of NADPH which would be measured, not the appearance of NADH as in the other schemes discussed above. This system is described in detail by D. M. Gibson and R. A. Parker in The Enzymes, XVIII, 179 (1987).

An additional preferred embodiment utilizes phenylalanine hydroxylase (PH) as the target protein. The regulatory enzyme is cAMP-dependent protein kinase. After the competitive binding reaction, said free conjugate is utilized by said kinase to phosphorylate the target protein, the result being an increase in enzyme activity:

$$\text{Ag-ATP} + \text{PH} \xrightarrow{\text{cAMP-dependent protein kinase}} \text{Ag-ADP} + \text{PH-P}$$

The reaction mediated by PH is shown below. One additional coupling enzyme is required in the signal generating sequence to convert the signal precursor into the signal material, NADH:

$$O_2 + \text{Pheynlalanine} + \text{Pterine} \xrightarrow{\text{PH-P}} H_2O + \text{tyrosine} + \text{dihydropterin}$$

$$\text{Dihydropterin} + \text{NAD} \xrightarrow[\text{Reductase}]{\text{Dihydropterin}} \text{Pterine} + \text{NADH}$$

Two other hydroxylases may be substituted for the above-mentioned target protein. These are tyrosine hydroxylase and tryptophan hydroxylase. These systems are described in detail by S. Kaufman in The Enzymes, XVIII, 217 (1987).

An additional preferred embodiment utilizes qlutamine synthetase (GS) as the target protein. The regulatory enzyme is adenylytransferase (AT). In this embodiment, the adenyl group from the ATP conjugate is transferred to the target protein:

$$\text{Ag-ATP} + \text{GS} \xrightarrow{\text{AT}} \text{Ag-AMP-GS} + PP_i$$

The attachment of the adenyl group to glutamine synthetase reduces the enzyme activity by about 95%. Thus, reactivity would be inversely proportional to antigen concentration. The reaction mediated by the target protein is the following:

$$NH^{4+} + \text{Glutamic Acid} \xrightarrow{\text{GS}} \text{Glutamine}$$

The signal-producing material is the ammonium ion, which is a reaction substrate. The concentration of this ion can be monitored with an ammonium electrode. This system is described in detail by H. Holzer and W. Duntze in Ann. Rev. Biochem., 40, 756 (1971).

The following working examples describe experiments which were performed in developing the present

invention. Standard commercially available reagent grade chemicals were used whenever possible. These examples are to be considered illustrative of the present invention and should not be interpreted as limiting its scope.

## EXAMPLE 1

## PREPARATION OF ATP-PHENYTOIN CONJUGATE

The synthesis of this conjugate involved acylation of the ribose moiety of ATP with a valeric acid derivative of phenytoin in anhydrous pyridine. Since the sodium salt of ATP was not soluble in pyridine, it was necessary to covert it into a form which was soluble.

First, ATP was converted into a pyridinium salt as follows: ATP sodium salt (1.1 g; 0.2 mmoles) was dissolved in 4 ml of water and passed through a 2.5x25 cm column of Dowex 50x2 (pyridinium form). The column was eluted with water (40 ml) until the eluent was free of uv absorbing material. The aqueous solution was lyophilized, and the resulting white solid was stored at -20° C. The product gave a single spot in TLC on cellulose plates (Eastman Kodak, Rochester, NY), using isobutyric acid-concentrated ammonium hydroxide - water (66:2:33).

Second, ATP was converted into a trioctylamine salt as follows. A clear solution of ATP-pyridinium salt (0.1 mmole) in methanol containing 250 ul of trioctylamine was evaporated to dryness under reduced pressure. The residue was dried by repeated evaporation from anhydrous pyridine and then from dry dimethylformamide. The oily residue was dissolved in 5 ml of dry pyridine.

Acylation with phenytoin-valeric acid: The pyridine solution containing ATP was cooled to 4° C and stirred with phenytoin-valeric acid (704 mg; 2 mmole) and dicyclohexylcarbodimide (206 mg; 0.1 mmole). After storing the solution for about 50 hours at 4° C, 2 ml of cold water was added and the solution was stored 4 more hours at 4° C. The purpose of this step was to decompose any mixed anhydride which may have formed. TLC of this reaction mixture was carried out on cellulose plates in isobutyric acid-ammonium hydroxide-water (66:1:16.5). The presence of phosphate was determined by spraying the plate with ammonia molybdate-water-70% perchloric acid-conc. HCl-acetone (1:10:5:2.2:82). The presence of adenosine was detected with an ultraviolet lamp. Two products were found which contained both phosphate and adenosine. The Rf values of these products were 0.45 and 0.60.

The reaction mixture was treated further as follows. Precipitated material was removed by centrifugation, and the supernatant was evaporated to dryness. The residue was partitioned between 10 ml of chloroform and 10 ml of water. The aqueous phase was rewashed with chloroform and adjusted to pH 7 with solid $NaHCO_3$. Product A (Rf 0.45) was extracted into the aqueous phase, and Product B (Rf 0.60) was extracted into the organic phase.

Both products had activity with phosphorylase kinase. Product A also interacted with antibody to phenytoin, which made it useful for a homogeneous immunoassay. Product B failed to interact with antibody; therefore, it was not useful for the immunoassay.

The conjugate (Product A) was further purified first by paper chromatogrpahy and then by Dowex ion exchange. Paper chromatography was carried out in ammonium sulfate-water-isopropanol (6:100:0.2 w/v/v). In this solvent, any remaining, unreacted ATP moved away from the origin, while the product did not move. The conjugate was eluted from the origin with water and unreacted ATP moved away from the origin, while the product did not move. The conjugate was eluted from the origin with water and lyophilized. Next, the conjugate was applied to a Dowex Ag1x8 column, chloride form. The column was washed with 10 mM HCl, then with 0.2 M NaCl containing 10 mM HCl to remove any remaining traces of ATP. Then the conjugate was eluted with 50% ethanol in 0.2 M NaCl containing 10 mM HCl. The use of ethanol desorbs hydrophobically bound materials, such as the phenytoin of the conjugate.

A possible structure for this material is shown below (IV). The hapten may be attached at either the 2′ or 3′ position on the ribose ring. It would be expected that other synthetic schemes leading to a conjugate in which the hapten is attached to the ribose ring by a different linkage or bond would be similarly suitable for use in the present invention.

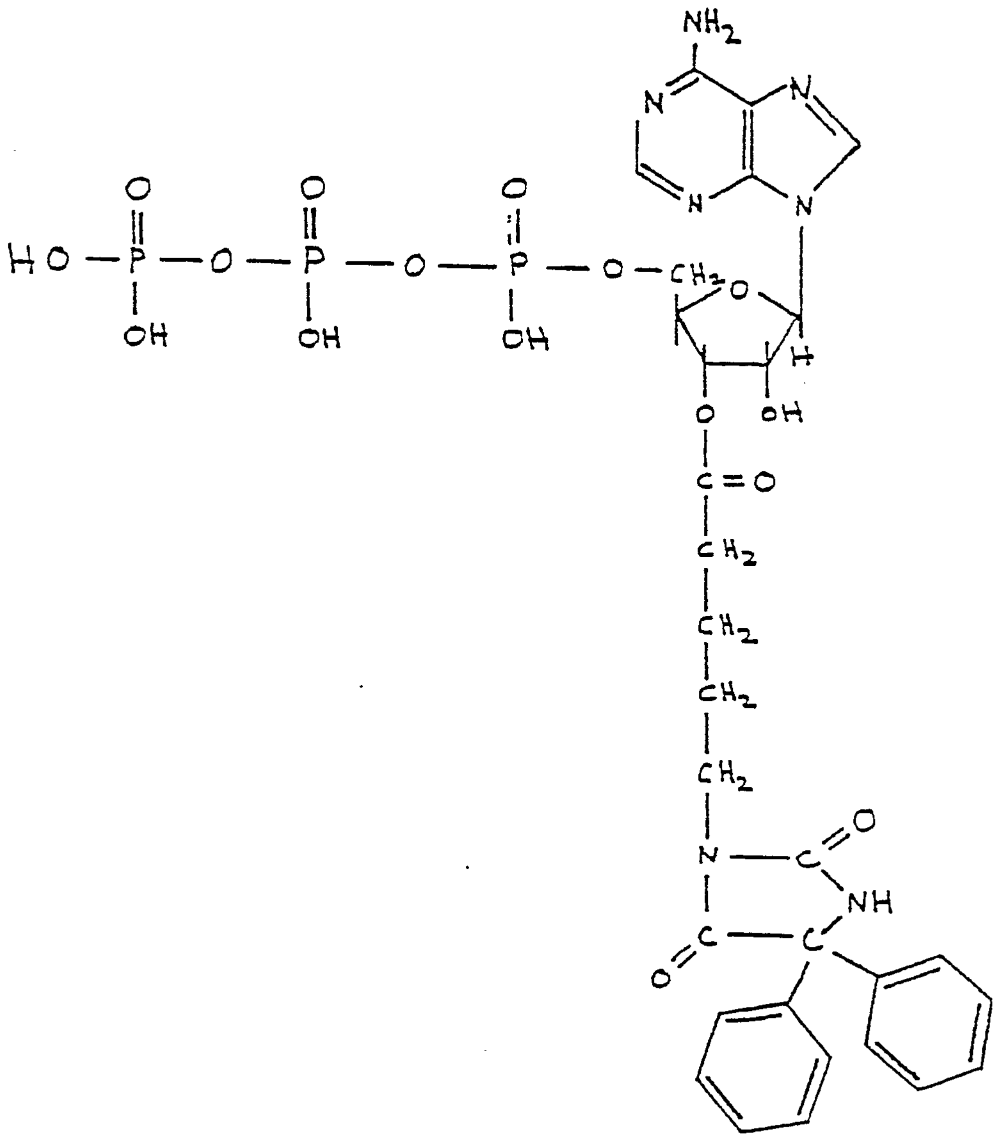

## EXAMPLE 2

## PREPARATION OF PHOSPHORYLASE KINASE

The regulatory enzyme was phosphorylase kinase. It was purified essentially as described by Cohen, P., Methods Enzymol. 99, 243-250 (1983), except that the tissue was obtained in frozen form from Pel Freeze from rabbits killed with thorazine instead of barbital. Also, the frozen tissue was ground in a mortar and pestle on dry ice to help prevent glycolysis, which resulted in higher yields of enzyme. The specific activity of the purified enzyme, after recovery from a Sepharose 4B column, was 11 µmoles/min/mg.

Units of phosphorylase kinase activity was assayed essentially as described by M. King and G. Carlson, Biochem., 20, 4282 (1981). The assay required two separate incubation mixtures. In the first, phosphorylase kinase was incubated with ATP and phosphorylase b. Then an aliquot was taken and diluted into a second reaction mixture, which measured the amount of product (phosphorylase a) formed in the first incubation. The first incubation mixture contained the following components: $6x10^{-5}$ M EDTA, $1.5x10^{-4}$ M DDT, $2x10^{-4}$

M $CaCl_2$, $1x10^{-2}$ M magnesium acetate, $4.2x10^{-2}$ M Tris buffer (pH 8.2 at 30°C), 60 mM β-glycerol phosphate, $3x10^{-2}$ M ATP, 2 mg/ml phosphorylase b, and an aliquot of a solution containing phosphorylase kinase. The second incubation mixture contained 2 mg/ml glycogen, 50 mM phosphate (pH 6.8), $4x10^{-5}$ M glucose 1,6-diphosphate, $1x10^{-2}$ M $MgCl_2$, $3.4x10^{-4}$ M NADP, 0.8 U/ml phosphoglucomutase, 6 U/ml glucose 6-phosphate dehydrogenase, and an aliquot of the first incubation mixture which contained phosphorylase a.

Materials: Phosphorylase b, glycogen, and other components of the reaction mixture were obtained from Sigma Chemical Co., St. Louis, MO. Sepharose 4B was obtained from Pharmacia Fine Chemicals, Piscataway, NJ. Dowex Ag1x8 was obtained from BioRad, Richmond, CA.

## EXAMPLE 3

## REACTION MIXTURE COMPOSITION FOR HOMOGENEOUS ASSAY

In order to carry out a homogeneous assay, the two separate reaction mixtures described above for the measurement of units of phosphorylase kinase were combined into a single reaction mixture. The blank reaction of phosphorylase b was found to be a major problem. This reaction was 2-3% of the magnitude of the reaction carried out by phosphorylase a. Since phosphorylase b was a substrate for phosphorylase kinase, a high concentration of this enzyme was required in the reaction mixture. This resulted in an unacceptably high blank reaction. When the pH was raised from 6.8 to 7.7 and 6 mM glucose was added to the reaction mixture, the blank reaction was twenty times lower. Glucose allosterically converted the more active R form of phosphorylase b into the less active T form. The blank reaction was reduced another 10x by passing a solution of phosphorylase b in 1 mM dithiothreitol through a column of Dowex Ag1x8, acetate form. This removed traces of AMP from the enzyme preparation (AMP is an allosteric activator of phosphorylase b). The blank reaction was reduced another two times by treating the glucogen solution with Dowex Ag1x8, hydroxyl form. As with phosphorylase b, traces of AMP were removed.

The composition of the reaction mixture for the one-step assay contained the following components: 4-6 U/ml phosphorylase kinase, $2x10^{-5}$ M phosphorylase b, 0.5 U/ml phosphoglucomutase, 0.5 U/ml glucose 6-phosphate dehydrogenase, 2.5 mg/ml glycogen, 20 mM potassium phosphate, $2.5x10^{-4}$ M NADP, 10 mM $MgCl_2$, 0.3 mM $CaCl_2$, 1 mM dithiothreitol, and 40 mM Tricine-HCl (pH 7.7).

Sensitivity: Using this reaction mixture, concentrations of ATP as low as $10^{-8}$ M were measured. The rate obtained at this concentration was about 10 mA/min. The conjugate was less active, and the limit of sensitivity was about $10^{-7}$ M.

Time courses of these reactions showed that the reaction rate was maximal after 15-20 minutes incubation at 30°C. At this time the scavenging of free ATP or conjugate was complete. This length of time was required, because the concentration of ATP was several orders of magnitude lower than Km of ATP for phosphorylase kinase (which was 0.3 mM).

## EXAMPLE 4

## IMMUNOREACTIVITY OF ATP-PHENYTOIN CONJUGATE

For the inventive assay principle to operate properly in a homogeneous format, phosphorylase kinase must not be able to utilize the conjugate as a substrate when it is bound to an antibody. The results in Table 8 show that the activity of the conjugate was inhibited by increasing amounts of antibody in the reaction mixture. The antibody was a monoclonal obtained from Kallestad Laboratories, Chaska, MN. The reaction mixture composition was the same as that described in Example 3, with ATP-phenytoin conjugate

at a concentrate of $3.6x10^{-6}$ M. The antibody and conjugate were incubated together in the reaction mixture 3-4 minutes prior to the addition of the enzymes. The rate was measured between 15 and 20 minutes after the addition of the enzymes. No inhibition was seen with unmodified ATP. Therefore, the immunoreactivity was specifically attributable to the phentyoin in the conjugate.

TABLE 8

| Antibody Added (µl) | Activity (mA/5min) |
|---|---|
| 0 | 63 |
| 30 | 42 |
| 45 | 29 |
| 60 | 21 |
| 80 | 17 |

EXAMPLE 5

DOSE RESPONSE TO PHENYTOIN

When the concentration of phenytoin was varied, the result was a dose response curve (Table 9). The reaction mixture was nearly the same as that described in Example 3 with slight further optimization. The antibody (60 µl/ml of reaction mixture) was incubated for 5 minutes with the indicated concentration of phenytoin. Then the conjugate was added, and an additional 5 minutes incubation was carried out. After this, the enzymes were added, and the measurement of reaction rate was begun. The rate was measured between 15 and 20 minutes after the addition of the enzymes.

TABLE 9

| Phenytoin (µM) | Activity (mA/5min) |
|---|---|
| 0 | 82 |
| 1.6 | 84 |
| 2.4 | 101 |
| 3.1 | 107 |
| 4.6 | 112 |
| 4.7 | 130 |
| 6.2 | 153 |

The dose response curve was also run using serum samples which had been fortified with phenytoin.

The concentrations of phenytoin were chosen to cover the clinically important range. This was 0 to 125 µM; or, 0 to 32 µg/ml. The samples were diluted by a factor of 18 into the reaction mixture. The protocol was equivalent to that described above, with two differences. First, the conjugate was purified both by TLC and by Dowex chromatography, whereas the conjugate in the previous experiment had been purified only by TLC. The more pure conjugate was twice as active as the less pure conjugate. Second, the amount of antibody used in the assay was 70 µl, not 60 µl. The results shown in Table 10 are essentially equivalent to those obtained with aqueous samples.

TABLE 10

| Phenytoin (µM) | Rate (mA/5min) |
|---|---|
| 0 | 153 |
| 1.3 | 166 |
| 2.8 | 168 |
| 3.8 | 179 |
| 4.8 | 185 |
| 6.1 | 200 |
| 6.9 | 209 |

It should be understood by those skilled in the art that various modifications may be made in the present invention without departing from the spirit and scope thereof as described in the specification and defined in the amended claims.

**Claims**

1. A test composition for use in a specific binding assay for detecting an analyte, comprising:

(a) a target protein capable of mediating a signal generating process, the mediating activity of said target protein being modified by the covalent bonding of a regulatory group thereto;

(b) a regulatory enzyme capable of catalyzing the covalent bonding of the regulatory group to said target protein;

(c) conjugate of a substrate molecule for the regulatory enzyme and an analyte which is the same as that under assay, said substrate containing said regulatory group;

(d) a specific binding partner for said analyte; and

(e) reagent means responsive to the condition of said target protein for generating a detectable signal.

2. A composition according to claim 1, wherein said specific binding partner is an antibody and the analyte is either an antigen or hapten.

3. A composition according to claim 1 or 2, wherein said target protein is an enzyme.

4. A composition according to claim 3, wherein said regulatory enzyme is phosphorylase kinase and said target protein is glycogen phosphorylase; or said regulatory enzyme is cyclic AMP-dependent protein kinase and said target protein is glycogen synthetase; or said regulatory enzyme is adenyltransferase and said target protein is glutamine synthetase; or said regulatory enzyme is branched chain ketoacid dehydrogenase kinase and said target enzyme is branched chain ketoacid dehydrogenase; or said regulatory enzyme is cyclic AMP-dependent protein kinase and said target protein is hormone sensitive lipase; or said regulatory enzyme is hydroxymethylglutaryl-CoA reductase kinase and said target protein is hydroxymethylglutaryl-CoA reductase; or said regulatory enzyme is cyclic AMP-dependent protein kinase

and said target protein is selected from phenylalanine hydroxylase, tyrosine hydroxylase and tryptophan hydroxylase; or said regulatory enzyme is adenyl transferase and said target protein is glutamine synthetase.

5. A method of detecting the presence and amount of an analyte in a sample, comprising:

(a) providing a regulatory enzyme and a target protein, the target protein being capable of mediating a process which yields a detectable signal, and the regulatory enzyme being capable of catalyzing the covalent bonding of a regulatory group to the target protein whereby the rate of the process mediated by the target protein is altered;

(b) providing a conjugate comprising the analyte covalently bonded to a substrate molecule for the regulatory enzyme and containing said regulatory group;

(c) providing a specific binding partner of the analyte, said specific binding partner being capable of binding to said conjugate, whereby said conjugate is restricted from acting as a substrate for the regulatory enzyme;

(d) contacting said conjugate with the specific binding partner of the analyte in the presence of the regulatory enzyme, the target protein, the elements of the process mediated by the target protein, and said sample;

(e) measuring the rate of the process mediated by the target protein; and

(f) comparing the rate of said process conducted in the presence of said sample with the rate of said process when conducted in the presence of a series of standard compositions containing known amounts of analyte.

6. A method according to claim 5, wherein said signal generating process is inhibited by said target protein upon the covalent bonding of said regulatory group thereto.

7. A method according to claim 5, wherein said signal generating process is stimulated by said target protein upon the covalent bonding of said regulatory group thereto.

8. A method according to claim 5, wherein said measurement step monitors the production of the reduced form of nicotinamide adenine dinucleotide phosphate.

9. A method according to claim 5,6,7 or 8, wherein said target protein is an enzyme.

10. A method according to any of claims 5 to 9, wherein said regulatory enzyme is phosphorylase kinase and said target protein is glycogen phosphorylase; or said regulatory enzyme is cyclic AMP-dependent protein kinase and said target protein is glycogen synthetase; or said regulatory enzyme is adenyl transferase and said target protein is glutamine synthetase; or said regulatory enzyme is branched chain ketoacid dehydrogenase kinase and said target enzyme is branched chain ketoacid dehydrogenase; or said regulatory enzyme is a cyclic AMP-dependent protein kinase and said target protein is hormone sensitive lipase; or said regulatory enzyme is hydroxymethylglutaryl-CoA reductase kinase and said target protein is hydroxymethylglutaryl-CoA reductase; or said regulatory enzyme is cyclic AMP-dependent protein kinase and said target protein is selected from phenylalanine hydroxylase, tyrosine hydroxylase and tryptophan hydroxylase; or said regulatory enzyme is adenyl transferase and said target protein is glutamine synthetase.